# EUROPEAN PATENT SPECIFICATION

(11) **EP 2 046 821 B1**
(45) Date of publication and mention of the grant of the patent: **18.05.2011**
(21) Application number: 07786341.3
(22) Date of filing: 25.07.2007
(51) Int. Cl.: C07K 14/47, A61K 38/17

(54) **FUSION PEPTIDE FOR INHIBITING INTERACTION OF NEURONAL NMDA RECEPTOR (NMDAR) AND NMDAR INTERACTING PROTEINS**
FUSIONPEPTID ZUM INHIBIEREN DER WECHSELWIRKUNG DES NEURONALEN NMDA-REZEPTORS (NMDAR) MIT NMDAR-WECHSELWIRKENDEN PROTEINEN
PEPTIDE DE FUSION PERMETTANT D'INHIBER L'INTERACTION ENTRE LE RÉCEPTEUR NEURONAL DU NMDA (NMDAR) ET LES PROTÉINES QUI INTERAGISSENT AVEC LE NMDAR

(30) Priority: 31.07.2006 EP 06015911
(43) Date of publication of application: 15.04.2009
(73) Proprietor: Xigen S.A., 1003 Lausanne (CH)
(72) Inventor: MEYER, Thomas, CH-4528 Zuchwil (CH)
(74) Representative: Graf von Stosch, Andreas
(86) International application number: PCT/EP2007/006619
(87) International publication number: WO 2008/014917

(56) References cited:
- WO-A-98/23781
- WO-A2-2004/045535
- US-A1- 2005 059 597
- YAMAMOTO YOKO ET AL: "Molecular design of bioconjugated cell adhesion peptide with a water-soluble polymeric modifier for enhancement of antimetastatic effect" CURRENT DRUG TARGETS, vol. 3, no. 2, April 2002 (2002-04), pages 123-130, XP002403767 ISSN: 1389-4501
- AARTS MICHELLE ET AL: "Treatment of ischemic brain damage by perturbing NMDA receptor- PSD-95 protein interactions." SCIENCE (NEW YORK, N.Y.) 25 OCT 2002, vol. 298, no. 5594, 25 October 2002 (2002-10-25), pages 846-850, XP002456870 ISSN: 1095-9203
- BORSELLO T ET AL: "A peptide inhibitor of c-Jun N-terminal kinase protects against excitotoxicity and cerebral ischemia" NATURE MEDICINE, NATURE PUBLISHING GROUP, NEW YORK, NY, US, vol. 9, no. 9, 24 August 2003 (2003-08-24), pages 1180-1186, XP002366119 ISSN: 1078-8956
- BORSELLO T ET AL: "Use of cell-permeable peptides to prevent neuronal degeneration" TRENDS IN MOLECULAR MEDICINE, ELSEVIER CURRENT TRENDS, vol. 10, no. 5, May 2004 (2004-05), pages 239-244, XP002366120 ISSN: 1471-4914

### SEQUENCE LISTING

<110> Xigen S.A.
<120> Fusion peptide for inhibiting interaction of neuronal NMDA receptor (NMDAR) and NMDAR interacting proteins
<130> AI02P003WO
<160> 35
<170> Patent In version 3.3
<210> 1
   <211> 16
   <212> PRT
   <213> Artificial
<220>
   <223> Description of sequence: transporter peptide pAntp (43-58) from Antenna pedia
<400> 1
   Arg Gln Ile Lys Ile Trp Phe Gln Asn Arg Arg Met Lys Trp Lys Lys
   1               5                   10                  15
<210> 2
   <211> 16
   <212> PRT
   <213> Artificial
<220>
   <223> Description of sequence: transporter peptide pAntp from Antennapedia (43-58)
<220>
   <221> MOD RES
   <222> (16)..(16)
   <223> AMIDATION
<400> 2
   Arg Gln Ile Lys Ile Trp Phe Gln Asn Arg Arg Met Lys Trp Lys Lys
   1               5                   10                  15
<210> 3
   <211> 10
   <212> PRT
   <213> Artificial
<220>
   <223> Description of sequence: transporter peptide derived from (HIV) TAT
<400> 3
   Gly Arg Lys Lys Arg Arg Gln Arg Arg Arg
   1               5                   10
<210> 4
   <211> 11
   <212> PRT
   <213> Artificial
<220>
   <223> Description of sequence: transporter peptide derived from (HIV) TAT
<400> 4
   Tyr Gly Arg Lys Lys Arg Arg Gln Arg Arg Arg
   1               5                   10
<210> 5
   <211> 15
   <212> PRT
   <213> Artificial
<220>
   <223> Description of sequence: transporter peptide derived from (HIV) TAT
<400> 5
   Cys Gly Arg Lys Lys Arg Arg Gln Arg Arg Arg Pro Pro Gln Cys
   1               5                   10                  15
<210> 6
   <211> 16
   <212> PRT
   <213> Artificial
<220>
   <223> Description of sequence: transporter peptide derived from (HIV) TAT
<400> 6
   Cys Gly Arg Lys Lys Arg Arg Gln Arg Arg Arg Pro Pro Gln Cys Cys
   1 5 10 15
<210> 7
   <211> 24
   <212> PRT
   <213> Artificial
<220>
   <223> Description of sequence: transporter peptide hCT(9-32)
<220>
   <221> MOD_RES
   <222> (24)..(24)
   <223> AMIDATION
<400> 7
   Leu Gly Thr Tyr Thr Gln Asp Phe Asn Lys Phe His Thr Phe Pro Gln
   1 5 10 15
   Thr Ala Ile Gly Val Gly Ala Pro
   20
<210> 8
   <211> 18
   <212> PRT
   <213> Artificial
<220>
   <223> Description of sequence: transporter peptide pVEC
<220>
   <221> MOD_RES
   <222> (18)..(18)
   <223> AMIDATION
<400> 8
   Leu Leu Ile Ile Leu Arg Arg Arg Ile Arg Lys Gln Ala His Ala His
   1 5 10 15
   Ser Lys
<210> 9
   <211> 16
   <212> PRT
   <213> Artificial
<220>
   <223> Description of sequence: transporter peptide pISL
<220>
   <221> MOD RES
   <222> (16)..(16)
   <223> AMIDATION
<400> 9
   Arg Val Ile Arg Val Trp Phe Gln Asn Lys Arg Cys Lys Asp Lys Lys
   1 5 10 15
<210> 10
   <211> 28
   <212> PRT
   <213> Artificial
<220>
   <223> Description of sequence: transporter peptide Mouse PRP (1-28)
<220>
   <221> MOD_RES
   <222> (28)..(28)
   <223> AMIDATION
<400> 10
   Met Ala Asn Gly Leu Tyr Trp Leu Leu Ala Leu Phe Val Thr Met Trp
   1 5 10 15
   Thr Asp Val Gly Leu Cys Lys Lys Arg Pro Lys Pro
   20 25
<210> 11
   <211> 27
   <212> PRT
   <213> Artificial
<220>
   <223> Description of sequence: transporter peptide Ems (194-220)
<220>
   <221> MOD_RES
   <222> (27)..(27)
   <223> AMIDATION
<400> 11
   Arg Gln Gly Ala Ala Arg Val Thr Ser Trp Leu Gly Leu Gln Leu Arg
   1 5 10 15
   Ile Ala Gly Lys Arg Leu Glu Gly Arg Ser Lys
   20 25
<210> 12
   <211> 13
   <212> PRT
   <213> Artificial
<220>
   <223> Description of sequence: transporter peptide Restricocin L3 (60-73)
<220>
   <221> MOD_RES
   <222> (13)..(13)
   <223> AMIDATION
<400> 12
   Lys Leu Ile Lys Gly Arg Thr Pro Ile Lys Phe Gly Lys
   1 5 10
<210> 13
   <211> 26
   <212> PRT
   <213> Artificial
<220>
   <223> Description of sequence: transporter peptide VT5
<220>
   <221> MOD_RES
   <222> (26)..(26)
   <223> AMIDATION
<400> 13
   Asp Pro Lys Gly Asp Pro Lys Gly Val Thr Val Thr Val Thr Val Thr
   1 5 10 15
   Val Thr Gly Lys Gly Asp Pro Lys Pro Asp
   20 25
<210> 14
   <211> 18
   <212> PRT
   <213> Artificial
<220>
   <223> Description of sequence: transporter peptide MAP
<220>
   <221> MOD_RES
   <222> (18)..(18)
   <223> AMIDATION
<400> 14
   Lys Leu Ala Leu Lys Leu Ala Leu Lys Ala Leu Lys Ala Ala Leu Lys
   1 5 10 15
   Leu Ala
<210> 15
   <211> 7
   <212> PRT
   <213> Artificial
<220>
   <223> Description of sequence: transporter peptide (Arg)7
<400> 15
   Arg Arg Arg Arg Arg Arg Arg
   1 5
<210> 16
   <211> 7
   <212> PRT
   <213> Artificial
<220>
   <223> Description of sequence: transporter peptide (Arg)7-NH2
<220>
   <221> MOD_RES
   <222> (7)..(7)
   <223> AMIDATION
<400> 16
   Arg Arg Arg Arg Arg Arg Arg
   1               5
<210> 17
   <211> 8
   <212> PRT
   <213> Artificial
<220>
   <223> Description of sequence: transporter peptide (Arg)7-C
<400> 17
   Arg Arg Arg Arg Arg Arg Arg Cys
   1               5
<210> 18
   <211> 8
   <212> PRT
   <213> Artificial
<220>
   <223> Description of sequence: transporter peptide (Arg)8
<400> 18
   Arg Arg Arg Arg Arg Arg Arg Arg
   1               5
<210> 19
   <211> 8
   <212> PRT
   <213> Artificial
<220>
   <223> Description of sequence: transporter peptide (Arg)8-NH2
<220>
   <221> MOD_RES
   <222> (8).. (8)
   <223> AMIDATION
<400> 19
   Arg Arg Arg Arg Arg Arg Arg Arg
   1               5
<210> 20
   <211> 9
   <212> PRT
   <213> Artificial
<220>
   <223> Description of sequence: transporter peptide (Arg)9
<400> 20
   Arg Arg Arg Arg Arg Arg Arg Arg Arg
   1               5
<210> 21
   <211> 9
   <212> PRT
   <213> Artificial
<220>
   <223> Description of sequence: transporter peptide (Arg)9-NH2
<220>
   <221> MOD_RES
   <222> (9)..(9)
   <223> AMIDATION
<400> 21
   Arg Arg Arg Arg Arg Arg Arg Arg Arg
   1               5
<210> 22
   <211> 27
   <212> PRT
   <213> Artificial
<220>
   <223> Description of sequence: transporter peptide MPG
<400> 22
   Gly Ala Leu Phe Leu Gly Phe Leu Gly Ala Ala Gly Ser Thr Met Gly
   1               5                   10                  15
   Ala Trp Ser Gln Pro Lys Ser Lys Arg Lys Val
               20                  25
<210> 23
   <211> 27
   <212> PRT
   <213> Artificial
<220>
   <223> Description of sequence: transporter peptide MPG
<220>
   <221> MOD RES
   <222> (27)..(27)
   <223> cysteamide modification
<400> 23
   Gly Ala Leu Phe Leu Gly Phe Leu Gly Ala Ala Gly Ser Thr Met Gly
   1 5 10 15
   Ala Trp Ser Gln Pro Lys Ser Lys Arg Lys Val
   20 25
<210> 24
   <211> 27
   <212> PRT
   <213> Artificial
<220>
   <223> Description of sequence: transporter peptide MPG-NH2
<220>
   <221> MOD_RES
   <222> (27)..(27)
   <223> AMIDATION
<400> 24
   Gly Trp Thr Leu Asn Ser Ala Gly Tyr Leu Leu Gly Lys Ile Asn Leu
   1 5 10 15
   Lys Ala Leu Ala Ala Leu Ala Lys Lys Ile Leu
   20 25
<210> 25
   <211> 21
   <212> PRT
   <213> Artificial
<220>
   <223> Description of sequence: transporter peptide Transportan 10
<220>
   <221> MOD RES
   <222> (21)..(21)
   <223> AMIDATION
<400> 25
   Ala Gly Tyr Leu Leu Gly Lys Ile Asn Leu Lys Ala Leu Ala Ala Leu
   1 5 10 15
   Ala Lys Lys Ile Leu
   20
<210> 26
   <211> 21
   <212> PRT
   <213> Artificial
<220>
   <223> Description of sequence: transporter peptide Pep-1
<400> 26
   Lys Glu Thr Trp Trp Glu Thr Trp Trp Thr Glu Trp Ser Gln Pro Lys
   1               5                   10                  15
   Lys Lys Arg Lys Val
               20
<210> 27
   <211> 21
   <212> PRT
   <213> Artificial
<220>
   <223> Description of sequence: transporter peptide Pep-1
<220>
   <221> MOD_RES
   <222> (21)..(21)
   <223> CYSTEAMIDE
<400> 27
   Lys Glu Thr Trp Trp Glu Thr Trp Trp Thr Glu Trp Ser Gln Pro Lys
   1               5                   10                  15
   Lys Lys Arg Lys Val
               20
<210> 28
   <211> 30
   <212> PRT
   <213> Artificial
<220>
   <223> Description of sequence: transporter peptide KALA
<400> 28
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 29
   <211> 21
   <212> PRT
   <213> Artificial
<220>
   <223> Description of sequence: transporter peptide Bulforin 2
<400> 29
   Thr Arg Ser Ser Arg Ala Gly Leu Gln Phe Pro Val Gly Arg Val His
   1 5 10 15
   Arg Leu Leu Arg Lys
   20
<210> 30
   <211> 9
   <212> PRT
   <213> Artificial
<220>
   <223> Description of sequence: NMDAR derived peptide (L-form)
<400> 30
   Lys Leu Ser Ser Ile Glu Ser Asp Val
   1 5
<210> 31
   <211> 9
   <212> PRT
   <213> Artificial
<220>
   <223> Description of sequence: NMDAR derived peptide (D-form)
<400> 31
   Val Asp Ser Glu Ile Ser Ser Leu Lys
   1 5
<210> 32
   <211> 20
   <212> PRT
   <213> Artificial
<220>
   <223> Description of sequence: L-TAT-L-NR2B9c sequence
<400> 32
   Tyr Gly Arg Lys Lys Arg Arg Gln Arg Arg Arg Lys Leu Ser Ser Ile
   1 5 10 15
   Glu Ser Asp Val
   20
<210> 33
   <211> 20
   <212> PRT
   <213> Artificial
<220>
   <223> Description of sequence: D-TAT-D-NR2B9c
<400> 33
   Val Asp Ser Glu Ile Ser Ser Leu Lys Arg Arg Arg Gln Arg Arg Lys
   1               5                   10                  15
   Lys Arg Gly Tyr
               20
<210> 34
   <211> 17
   <212> PRT
   <213> Artificial
<220>
   <223> Description of sequence: L-(Arg)8-L-NR2B9c
<400> 34
<210> 35
   <211> 17
   <212> PRT
   <213> Artificial
<220>
   <223> Description of sequence: D-(Arg)8-D-NR2B9c
<400> 35
   Val Asp Ser Glu Ile Ser Ser Leu Lys Arg Arg Arg Arg Arg Arg Arg
   1               5                   10                  15
   Arg

## Description

Ischemic or traumatic injuries to the brain or spinal cord such as ischemic cerebral stroke are major health problems as they occur frequently and often produce irreversible damage to central nervous system (CNS) neurons and to their processes. Since the damage is often severe, major expenses are currently spent by health authorities each year for treatment of patients suffering from the consequences of such injuries. However, at present there are still no effective pharmacological treatments for those acute CNS injuries.

Clinically, ischemic or traumatic injuries to the brain or spinal cord such as ischemic cerebral stroke manifest themselves as acute deteriorations in neurological capacity ranging from small focal defects to catastrophic global dysfunction or may even lead to death of the patient to be treated. It is currently felt that the final magnitude of the deficit is dictated by the nature and extent of the primary physical insult, and by a sequence of processes of evolving secondary phenomena which cause further death of involved neuronal cells. Since commitment to these processes is not instantaneous, there exists a theoretical time-window

The present invention provides a fusion peptide comprising at least a component (I), wherein component (I) comprises a cell membrane penetrating peptide, and a component (II), selected from a peptide comprising the sequence vdseisslk (SEQ. ID NO: 31) or a peptide comprising a sequence with a sequence identity of about 60, 70, 80, 90, 95 or even 99 % with the sequence of SEQ ID NO: 31, wherein component (II) entirely consists of D-enantiomeric amino acids, wherein component (I) directs the fusion peptide across the cell membrane and wherein component (II) is in the fusion peptide still capable to inhibit interaction of N-methyl-D-aspartate receptor (NMDAR) with NMDAR interacting proteins. The present invention furthermore provides an inventive pharmaceutical composition, comprising the inventive fusion peptide and methods for administering the same as well as kits and uses employing the inventive fusion peptide.

of a yet undetermined duration, in which a timely intervention might interrupt the events causing a delayed neurotoxicity. Although a lot is known about the cellular mechanisms triggering and maintaining the processes of ischemic or traumatic neuronal death, e.g. reversal of the glutamate transporter, there are presently no effective practical preventative strategies or effective treatment protocols. Consequently, there are currently no effective clinically useful pharmacological treatments available for the above diseases including cerebral stroke or spinal cord injury.

From a scientific view, a local reduction in CNS tissue perfusion mediates neuronal death in both hypoxic and traumatic CNS injuries *in vivo.* Such a local hypoperfusion is usually caused by a physical disruption of the local vasculature, vessel thrombosis, vasospasm, or luminal occlusion by an embolic mass. Regardless of its etiology, the resulting ischemia is believed to damage susceptible neurons by impacting adversely on a variety of cellular homeostatic mechanisms. Although the nature of the exact disturbances is poorly understood, a feature common to many experimental models of neuronal injury is a rise in free intracellular calcium concentration ([Ca²⁺]ᵢ). Neurons possess multiple mechanisms to confine [Ca²⁺]ᵢ to the low levels, about 100 nM necessary for the physiological function. It is widely believed that a prolonged rise in [Ca²⁺]ᵢ deregulates tightly-controlled Ca²⁺-dependent processes, causing them to yield excessive reaction products, to activate normally quiescent enzymatic pathways, or to inactivate regulatory cytoprotective mechanisms. This, in-turn, results in the creation of experimentally observable cell destruction, such as lipolysis, proteolysis, cytoskeletal breakdown, pH alterations, free radical formation, mitochondrial dysfunction, cellular swelling, loss of plasma integrity and nuclear pyknosis.

A key cause of harmful levels of Ca²⁺ entry during an ischemic episode is through excessive release of EAAs. Thus, the classical approach to prevent Ca²⁺ neurotoxicity has been through pharmacological blockade of Ca²⁺ entry through voltage-gated Ca²⁺ channels and/or of excitatory amino acid EAA-gated channels. Variations on this strategy often lessen EAA-induced or anoxic cell death *in vitro,* lending credence to the Ca²⁺-neurotoxicity hypothesis. However, a variety of Ca²⁺ channel- and EAA-antagonists fail to protect against neuronal injury *in vivo,* particularly in experimental Spinal Cord Injury (SCI), head injury and global cerebral ischemia. It is unknown whether this is due to insufficient drug concentrations, inappropriate Ca²⁺ influx blockade, or to a contribution from non-Ca²⁺ dependent neurotoxic processes. However, it is likely that Ca²⁺ neurotoxicity is triggered through different pathways in different CNS neuron types. Hence, successful Ca²⁺-blockade would typically require a polypharmaceutical approach. It is to be noted that many antagonists of NMDARs have been developed. However, those antagonists have been successful *in vivo* in animal models just upon administration of the drug prior to the trauma and thus may not be translated to a typical clinical scenario, wherein treatment typically occurs subsequent to e.g. a stroke. Also, NMDAR antagonists are typically poorly tolerated *in vivo* in humans, providing a small or sometimes even not existing therapeutic concentration window, which reflects the important role of NMDAR in normal physiology as well as in pathophysiology.

Regarding Ca²⁺ dependent neurotoxic processes it was observed in the mammalian nervous system, that the efficiency by which N-methyl-D-aspartate receptor (NMDAR) activity triggers intracellular signaling pathways governs neuronal plasticity, development, senescence and disease. Studying excitotoxic NMDAR signaling by suppressing the expression of the NMDAR scaffolding protein PSD-95 revealed a selectively attenuated NMDAR excitotoxicity, but, however, not excitotoxicity by other glutamate or Ca²⁺ channels (see e.g. US 20030050243). NMDAR function was thereby unaffected, as receptor expression, while NMDA-currents and ⁴⁵Ca loading via NMDARs were unchanged. Furthermore, suppressing PSD-95 selectively blocked Ca²⁺-activated nitric oxide production by NMDARs, but not by other pathways, without affecting neuronal nitric oxide synthase (nNOS) expression or function. Thus, PSD-95 was regarded to be necessary for efficient coupling of NMDAR activity to nitric oxide toxicity and imparts specificity to excitotoxic Ca²⁺ signaling.

Additionally, it is known that calcium influx through NMDARs plays a key role in mediating synaptic transmission, neuronal development, and plasticity (see e.g. Ghosh and Greenberg, Science 268, 239 (1995); Bliss and Collingridge, Nature 361, 31 (1993)). In excess, Ca²⁺ influx triggers excitotoxicity (see e.g. Olney, Kainic acid as a tool in neurobiology., McGeer, Olney and McGeer, Eds. (Raven Press, New York, 1978), p. 95.; Rothman and Olney, TINS 10, 299 (1987); Choi, Ann NY Acad Sci 747, 162 (1994)), a process that damages neurons in neurological disorders that include stroke, epilepsy, and chronic neurodegenerative conditions (see e.g. Lipton and Rosenberg, New Eng J Med 330, 613 (1994)). It is to be noted that rapid Ca²⁺-dependent neurotoxicity is triggered most efficiently when Ca²⁺ influx occurs through NMDARs, and cannot be reproduced by loading neurons with equivalent quantities of Ca²⁺ through non-NMDARs or voltage-sensitive Ca²⁺ channels (VSCCs) (see e.g. Sattler et al., J Neurochem 71, 2349 (1998).; Tymianski et al., J Neurosci 13, 2085 (1993)). This observation indicated that Ca²⁺ influx through NMDAR channels may be functionally coupled to neurotoxic signaling pathways. Thus, without being bound by theory, it was suggested that lethal Ca²⁺ signaling by NMDARs may be determined by the molecules with which they physically interact. E.g., the NR2 NMDAR subunits, through their intracellular C-terminal domains, bind to PSD-95/SAP90 (see Cho et al., Neuron 9, 929 (1992)), chapsyn-110/PSD-93, and other members of the membrane-associated guanylate kinase (MAGUK) family (see e.g. Kornau et al., Science 269, 1737 (1995).; Brenman et al., J Neurosci 16, 7407 (1996); Muller et al., Neuron 17, 255 (1996)). NMDAR-bound MAGUKs are generally distinct from those associated with non-NMDARs (see Dong et al., Nature 386, 279 (1997); Brakeman et al., Nature 386, 284 (1997)). It was found that the preferential activation of neurotoxic Ca²⁺ signals by NMDARs is determined by the distinctiveness of NMDAR-bound MAGUKs, or of the intracellular proteins that they bind. PSD-95 is a submembrane scaffolding molecule that binds and clusters NMDARs preferentially and, through additional protein-protein interactions, may link them to intracellular signaling molecules (see e.g. Craven and Bredt, Cell 93, 495 (1998); Niethammer et al., Neuron 20, 693 (1998); Kim et al., Neuron 20, 683 (1998); Tezuka et al., Proc Natl Acad Sci USA 96, 435(1999).). Perturbing PSD-95 was thus suggested to have an impact on neurotoxic Ca²⁺ signaling through NMDARs.

In general, protein-protein interactions govern the signals involved in cell growth, differentiation, and intercellular communication through dynamic associations between modular protein domains and their cognate binding partners (see Pawson and J. D. Scott, Science 278, 2075-2080 (1997)). More particularly, ionotropic glutamate receptors are organized at excitatory synapses of central neurons into multi-protein signaling complexes within the post-synaptic density (PSD) (see Sheng, Proc. Natl. Acad. Sci. U.S.A 98, 7058-7061 (2001)). A prominent organizing protein within the PSD is PSD-95, a member of the membrane-associated guanylate kinase (MAGUK) family. PSD-95 contains multiple domains that couple transmembrane proteins such as the N-methyl-D-aspartate subtype of glutamate receptors (NMDAR) to a variety of intracellular signaling enzymes. Through its second PDZ domain (PDZ2), PSD-95 binds both the NMDAR 2B subunit (NR2B) and neuronal nitric oxide synthase (nNOS) (see Brenman et al., Cell 84, 757-767 (1996)). This interaction couples NMDAR activity to the production of nitric oxide (NO), a signaling molecule that mediates NMDAR-dependent excitotoxicity (see Dawson et al., Proc Natl Acad Sci USA 88, 6368-6371 (1991). NR2 can interact with either PDZ1 or PDZ2 of PSD-95. However, if the PDZ1 domain of PSD-95 interacts with the COOH terminus of the NMDA receptor, PDZ2 is free to bind the NH2-terminal region of nNOS (Cao et al., 2005, J. Cell Biol 168, 117-126; and Christopherson et al, 2005, J. Biol. Chem. 274:27467-27473.

Although NMDARs play an important neurotoxic role in hypoxic/ischemic brain injury (see Simon et al., Science 226, 850-852 (1984)), blocking NMDAR function may be deleterious in animals and humans (see Fix et al., Exp Neurol 123, 204-215 (1993); Davis et al, Stroke 31, 347-354 (2000); Morris et al., J. Neurosurg. 91, 737-743 (1999)). NMDAR antagonists trigger apoptosis in the developing brain. Also, NMDAR antagonists, when administered during a critical period after traumatic brain injury or during slowly progressing neurodegeneration exacerbates neuronal loss in the adult brain (Ikonomidou et al., 2000, Proc Natl Acad Sci U S A 97, 12885-12890; Olney et al., 2002, Brain Pathology 12, 488-498). Furthermore, blocking NMDAR function by using NMDAR antagonists may lead to other undesirable side effects such as cognitive impairment and psychosis. Targeting PSD-95 protein therefore represents an alternative therapeutic approach for diseases that involve excitotoxicity that may circumvent the negative consequences of blocking NMDAR function. However, mutation or suppression of PSD-95 has been proven impractical as a therapy for brain injury and cannot be applied after an injury has occurred. Therefore, rather than alter PSD-95 expression, it was questioned whether interfering with the NMDAR/PSD-95 interaction could suppress excitotoxicity *in vitro* and ischemic brain damage *in vivo.*

In view of the above, an approach was developed in the art (US 20030050243) to reduce the damaging effect by treatment of mammalian cells with compounds which reduce or inhibit interaction of N-methyl-D-aspartate (NMDA) receptors and NMDAR interacting proteins. The method according to US 20030050243 uses peptide compounds capable to disrupt interaction of the neuronal N-methyl-D-aspartate receptors (NMDARs) and NMDAR interacting proteins, e.g. neuronal proteins. This approach circumvents disadvantages, which result from blockage of NMDAR function. The method according to US 20030050243 is mainly based on the finding that interaction of postsynaptic density-95 protein (PSD-95) to neuronal N-methyl-D-aspartate receptors (NMDARs) leads to pathways mediating excitotoxicity, and, hence, ischemic brain damage may be cured using selective peptides disrupting this particular interaction. More particularly, disruption of the interaction was accomplished according to US 20030050243 by introducing peptides into neurons that bind to modular domains on either side of the PSD-95/NMDAR interaction complex. This treatment attenuated downstream NMDAR signaling without blocking NMDAR activity, protected cultured cortical neurons from excitotoxic insults and reduced cerebral infarction volume in rats subjected to transient focal cerebral ischemia. The method of treatment according to US 20030050243 was effective when applied either before or one hour subsequent to onset of excitotoxicity *in vitro* and cerebral ischemia *in vivo.* This approach was thus shown to provide a good basis to prevent negative complications associated with blocking NMDAR activity.

However, it is well known that peptide compounds administered *in vivo* targeting intracellular sites, e.g. for treatment of a specific disease, as well as for *in vitro* cell treatment, typically comprise poor bioavailability in cells to be treated. This poor bioavailability is mainly due to a sub-optimal direction of these peptide compounds to their target cells. This may be caused e.g. by lack of suitable transporter systems or by use of non-adequate transporter systems, which do not efficiently work *in vivo* in the location of the desired target cell(s), e.g. which do not efficiently target the cell cytoplasm or a specific cellular destination. Though this effect may be overcome by administering an increased amount of peptide compounds or by repeated administration thereof, increased levels of peptide compounds *in vivo* may lead to undesired side effects upon administration. Furthermore, repeated administration of peptide compounds typically requires continuous presence of a medical doctor and, as a consequence, in most cases continued hospitalization. Even if such effects may be rendered moot by increased or repeated administration of peptide compounds or by using more adequate transporter systems (see e.g. US 20030050243, using TAT as a transporter peptide), they additionally may undergo a pretermed degradation and, as a consequence, show again poor bioavailability *in vivo.* This effect is in part due to intracellular processes, e.g. degradation processes by decomposition enzymes like proteases or peptidases, or may be due to *in vivo* instability of these peptides or proteolytic degradation in serum, cerobospinal fluid (csf), etc.. However, peptide instability is neither abolished by increased nor repeated administration of the peptide compounds.

Starting from the above, it was thus an object of the present invention to provide an alternative to reduce the damaging effect of an injury to mammalian cells, more particularly to provide compounds, which inhibit the interaction of neuronal N-methyl-D-aspartate (NMDA) receptor and NMDAR interacting proteins, thereby overcoming at least some of the prior art restrictions, e.g. as exemplified by compounds according to US 20030050243.

The inventive fusion peptide as defined above comprises as component (I) a transporter peptide. Such a transporter peptide may be selected from any transporter peptide that directs the inventive fusion peptide into the cell cytoplasm or, even further, to a specific intracellular destination. The transporter peptide used as component (I) can e.g. direct the inventive fusion peptide to a desired location within the cell, e.g., the nucleus, the ribosome, the endoplasmatic reticulum, lysosome, or peroxisome. Consequently, in a preferred embodiment the transporter peptide of the inventive fusion peptide directs the conjugate molecule to a defined cellular location. In any case, the transporter peptide used as component (I) of the inventive fusion peptide preferably directs the inventive fusion peptide across the plasma membrane, e.g. from the extracellular cell environment through the plasma membrane into the cytoplasma, thereby enhancing the cellular uptake of the inventive fusion peptide in particular by enhancing its cell permeability or by enhancing its intracellular retention time.

This object is solved according to the present invention by a fusion peptide comprising at least a component (I), wherein component (I) comprises a cell membrane penetrating peptide, and a component (II), selected from a peptide comprising the sequence vdseisslk (SEQ. ID NO: 31) or a peptide comprising a sequence with a sequence identity of about 60, 70, 80, 90, 95 or even 99 % with the sequence of SEQ ID NO: 31, wherein component (II) entirely consists of D-enantiomeric amino acids, wherein component (I) directs the fusion peptide across the cell membrane and wherein component (II) is in the fusion peptide still capable to inhibit interaction of N-methyl-D-aspartate receptor (NMDAR) with NMDAR interacting proteins. For the purpose of the present invention, D-enantiomeric amino acids are indicated by small letters in a sequence as indicated herein, whereas L-enantiomeric amino acids are indicated by capital letters.

More preferably, component (I) of the fusion peptide as defined above may be selected from cell membrane penetrating peptides comprising, without being limited thereto, (a) protein transduction domains (PTD) and protein derived CPPs, such as sequences derived from Antennapedia, e.g. pAntp (43-58), comprising the sequences RQIKIWFQNRRMKWKK (SEQ ID NO: 1) or RQIKIWFQNRRMKWKK-amide (SEQ ID NO: 2); or sequences derived from human immunodeficiency virus 1 (HIV-1), e.g. Tat, more preferably region 37 to 72, region 37 to 60, region 48 to 60 or region 49 to 57 from TAT, e.g. comprising the sequences GRKKRRQRRR (SEQ ID NO: 3), YGRKKRRQRRR (SEQ ID NO: 4), CGRKKRRQRRRPPQC (SEQ ID NO: 5) or CGRKKRRQRRRPPQCC (SEQ ID NO: 6); hCT(9-32), comprising the sequence LGTYTQDFNKFHTFPQTAIGVGAP-NH₂ (SEQ ID NO: 7); *p*VEC, comprising the sequence LLIILRRRIRKQAHAHSK-NH₂ (SEQ ID NO: 8); pISL, comprising the sequence RVIRVWFQNKRCKDKK-NH₂ (SEQ ID NO: 9); Mouse PRP (1-28), comprising the sequence MANGLYWLLALFVTMWTDVGLCKKRPKP-NH₂ (SEQ ID NO: 10) or its human homologs; E^{ms} (194-220), comprising the sequence RQGAARVTSWLGLQLRIAGKRLEGRSK-NH₂ (SEQ ID NO: 11); Restricocin L3 (60-73), comprising the sequence KLIKGRTPIKFGK-NH₂ (SEQ ID NO: 12); etc., (b) model peptides such as VT5 comprising the sequence DPKGDPKGVTVTVTVTVTGKGDPKPD-NH₂ (SEQ ID NO: 13), MAP comprising the sequence KLALKLALKALKAALKLA-NH₂ (SEQ ID NO: 14), arginine stretches including RRRRRRR, i.e. (Arg)₇ (SEQ ID NO: 15), or RRRRRRR-NH₂, i.e. (Arg)₇-NH₂ (SEQ ID NO: 16), or RRRRRRR-C, i.e. (Arg)₇-C (SEQ ID NO: 17), or RRRRRRRR, i.e. (Arg)₈ (SEQ ID NO: 18), or RRRRRRRR-NH₂, i.e. (Arg)₈-NH₂ (SEQ ID NO: 19), RRRRRRRRR, i.e. (Arg)₉ (SEQ ID NO: 20), or RRRRRRRRR-NH₂, i.e. (Arg)₉-NH₂, (SEQ ID NO: 21), etc., or (c) designed CPPs such as MPG comprising the sequence GALFLGFLGAAGSTMGAWSQPKSKRKV (SEQ ID NO: 22) or GALFLGFLGAAGSTMGAWSQPKSKRKV-cysteamide (SEQ ID NO: 23), Transportan having the sequence GWTLNSAGYLLGKINLKALMLAKKIL-NH₂ (SEQ ID NO: 24), Transportan 10 comprising the sequence AGYLLGKINLKALAALAKKIL-NH₂ (SEQ ID NO: 25), Pep-1 comprising the sequence KETWWETWWTEWSQPKKKRKV (SEQ ID NO: 26) or KETWWETVVVVTEWSQPKKKRKV-cysteamide (SEQ ID NO: 27), etc., or may be selected from the KALA peptide comprising the sequence WEAKLAKALAKALAKHLAKALAKALKACEA (SEQ ID NO: 28), from Bulforin 2 comprising the sequence TRSSRAGLQFPVGRVHRLLRK (SEQ ID NO: 29), etc.. Peptides used as component (I) of the inventive fusion peptide may furthermore be selected from peptide sequence having a sequence identity of about 60, 70, 80, 90, 95 or even 99 % with a peptide sequence according to any of SEQ ID NOs: 1 to 29 as defined above, provided that component (I) still retains its biological activity, i.e. to direct the inventive fusion peptide across the plasma membrane. Selection of a penetrating peptide as defined above as component (I) of the inventive fusion peptide will typically be carried out on the basis of the specific requirements of the cell system to which the inventive fusion peptide is to be administered, e.g. efficiency of transport in the specific cell system, membranes, etc.. Biological activity of a transporter peptide as shown above or as known in the art may be easily determined by a skilled person by using standard assays. E.g., a transporter peptide may be fused to a protein such as GFP, or may be labelled with a radioactive label, enzyme or fluorophore etc. which can be readily detected in a cell. Then, the fused transporter peptide is transfected into a cell or added to a culture supernatant and permeation of cell membranes can be monitored by using biophysical standard methods.

Component (I) of the fusion peptide as defined above may be composed either of naturally occurring amino acids, i.e. L-amino acids, or of D-amino acids, i.e. of an amino acid sequence comprising D-amino acids in retro-inverso order as compared to the native sequence. The term "retro-inverso" refers to an isomer of a linear peptide in which the direction of the sequence is reversed and the chirality of each amino acid residue is inverted. Thus, any sequence herein, being present in L-form is also inherently disclosed herein as a D-enantiomeric (retro-inverso) peptide sequence. D-enantiomeric (retro-inverso) peptide sequences according to the invention can be constructed, e.g. by synthesizing a reverse of the amino acid sequence for the corresponding native L-amino acid sequence. In D-retro-inverso enantiomeric peptides, e.g. a component of the inventive fusion peptide, the positions of carbonyl and amino groups in each single amide bond are exchanged, while the position of the side-chain groups at each alpha carbon is preserved. Retro-inverso peptides, if used as a component of the inventive fusion peptide, possess a variety of useful properties. For example, they enter cells more efficiently, are more stable (especially *in vivo*) and show lower immunogenicity than corresponding L-peptides. In contrast, naturally-occuring proteins typically contain L-amino acids. Therefore, almost all decomposition enzymes, like proteases or peptidases, cleave peptide bonds between adjacent L-amino acids. Consequently, inventive fusion peptides composed of D-enantiomeric amino acids in retro-inverso order, particularly for component (II) and, optionally, component (I), are largely resistant to proteolytic breakdown.

Preparation of a component of the inventive fusion peptide as defined above having D-enantiomeric amino acids can be achieved by chemically synthesizing a reverse amino acid sequence of the corresponding naturally occurring L-form amino acid sequence or by any other suitable method known to a skilled person. This synthesis is preferably carried out by solid phase synthesis linking D amino acids to the desired retro-inverso sequence. Apart from the D amino acids used and the synthesis of the amino acids in retro-inverso order the solid phase synthesis of the inventive D amino acid sequences is chemically identical with the synthesis of peptides on the basis of L amino acids. A general method for the construction of any desired DNA sequence is provided, e.g., in Brown J. et al. (1979), Methods in Enzymology, 68:109; Sambrook J, Maniatis T (1989), *supra.*

Alternatively, the D-retro-inverso-enantiomeric form of an inventive fusion peptide or a component thereof may be prepared using chemical synthesis as disclosed above utilizing an L-form of an inventive fusion peptide or a component thereof as a matrix for chemical synthesis of the D-retro-inverso-enantiomeric form.

Component (II) of the inventive fusion peptide as defined above is selected from a sequence comprising the D-enantiomeric amino acid sequence vdseisslk (SEQ ID NO: 31) or a sequence showing sequence identity of about 60, 70, 80, 90, 95, or most preferably 99 % with a sequence according to SEQ ID NO: 31.

The term "sequence identity" as defined herein means that the sequences are compared as follows. To determine the percent identity of two amino acid sequences, the sequences can be aligned for optimal comparison purposes (e.g., gaps can be introduced in the sequence of a first amino acid sequence). The amino acids at corresponding amino acid positions can then be compared. When a position in the first sequence is occupied by the same amino acid as the corresponding position in the second sequence, then the molecules are identical at that position. The percent identity between the two sequences is a function of the number of identical positions shared by the sequences. E.g., where a particular peptide is said to have a specific percent identity to a reference polypeptide of a defined length, the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Hence, the particular peptide said to have a specific percent identity will e.g. be selected, without being limited thereto, from a concrete peptide e.g. according to any of SEQ ID NOs: 1 to 29 for component (I) and from a concrete peptide e.g. according to SEQ ID NO: 31 for component (II) or from peptides e.g. according to SEQ ID NOs: 33 and 35 for the entire fusion peptide. Of course, other polypeptides will meet the same criteria. Such a determination of percent identity of two sequences can be accomplished using a mathematical algorithm. A preferred, non-limiting example of a mathematical algorithm utilized for the comparison of two sequences is the algorithm of Karlin et al. (1993), PNAS USA, 90:5873-5877. Such an algorithm is incorporated into the NBLAST program, which can be used to identify sequences having the desired identity to the amino acid sequence of the invention. To obtain gapped alignments for comparison purposes, Gapped BLAST can be utilized as described in Altschul et al. (1997), Nucleic Acids Res, 25:3389-3402. When utilizing BLAST and Gapped BLAST programs, the default parameters of the respective programs (e. g., NBLAST) can be used. The sequences further may be aligned using Version 9 of the Genetic Computing Group's GAP (global alignment program), using the default (BLOSUM62) matrix (values-4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the claimed sequence. The described methods of determination of the percent identity of two amino acid sequences can be applied correspondingly to nucleic acid sequences.

Component (II) of the inventive fusion peptide as defined above is composed entirely of D-enantiomeric amino acids, i.e. of an amino acid sequence comprising D-amino acids in retro-inverso order as defined above, and may be prepared as disclosed above. Retro-inverso peptides as used for component (II) of inventive fusion peptides possess a variety of useful properties as already described for component (I) above, e.g. longer bioavailability of the component forming a section of the inventive fusion peptide due to lack of proteolytic degradation of this component, etc..

Both components (I) and (II) of the inventive fusion peptide may be linked directly or via a linker. A "linker" in the present context is preferably an oligo- or polypeptide and may be used to link components (I) and (II) as defined above. Preferably, a linker has a length of 1-10 amino acids, more preferably a length of 1 to 5 amino acids and most preferably a length of 1 to 3 amino acids. Advantageously, the linker does not have any secondary structure forming properties, i.e. has no α-helix or β-sheet structure forming tendency, e.g. if the linker is composed of at least 35 % of glycin residues. A linker may be typically be an all-glycine sequence, e.g. GG, GGG, GGGG, GGGGG, etc.. The use of a(n) intracellularly/endogenously) cleavable oligo- or polypeptide sequence as a linker permits component (II) to separate from component (I) after delivery into the target cell. Cleavable oligo- or polypeptide sequences in this context also include protease cleavable oligo- or polypeptide sequences, wherein the protease cleavage site is typically selected dependent on the protease endogenously expressed by the treated cell. The linker as defined above, if present as a oligo- or polypeptide sequence, may be composed either of D-amino acids or of naturally occurring amino acids, i.e. L-amino acids. As an alternative to the above, coupling of components (I) and (II) of the inventive fusion peptide can be accomplished via a coupling or conjugating agent, e.g a cross-linking reagent. There are several intermolecular cross-linking reagents which can be utilized, see for example, Means and Feeney, Chemical Modification of Proteins, Holden-Day, 1974, pp. 39-43. Among these reagents are, for example, N-succinimidyl 3-(2-pyridyldithio)propionate (SPDP) or N,N'-(1,3-phenylene)bismaleimide; N,N'-ethylene-bis-(iodoacetamide) or other such reagent having 6 to 11 carbon methylene bridges; and 1,5-difluoro-2,4-dinitrobenzene. Other cross-linking reagents useful for this purpose include: p,p'-difluoro-m,m'-dinitrodiphenylsulfone; dimethyl adipimidate; phenol-1,4-disulfonylchloride; hexamethylenediisocyanate or diisothiocyanate, or azophenyl-p-diisocyanate; glutaraldehyde and disdiazobenzidine. Cross-linking reagents may be homobifunctional, i.e., having two functional groups that undergo the same reaction. A preferred homobifunctional cross-linking reagent is bismaleimidohexane (BMH). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of proteins (or polypeptides) that contain cysteine residues. Cross-linking reagents may also be heterobifunctional. Heterobifunctional cross-linking reagents have two different functional groups, for example an amine-reactive group and a thiol-reactive group, that will cross-link two proteins having free amines and thiols, respectively. Examples of heterobifunctional cross-linking reagents are succinimidyl 4-(N-maleimidomethyl) cyclohexane-1-carboxylate (SMCC), m-maleimidobenzoyl-N-hydroxysuccinimide ester (MBS), and succinimide 4-(p-maleimidophenyl)butyrate (SMPB), an extended chain analog of MBS. The succinimidyl group of these cross-linking reagents with a primary amine, and the thiol-reactive maleimide forms a covalent bond with the thiol of a cysteine residue. Because cross-linking reagents often have low solubility in water, a hydrophilic moiety, such as a sulfonate group, may be added to the cross-linking reagent to improve its water solubility. Sulfo-MBS and sulfo-SMCC are examples of cross-linking reagents modified for water solubility. Many cross-linking reagents yield a conjugate that is essentially non-cleavable under cellular conditions. Therefore, some cross-linking reagents contain a covalent bond, such as a disulfide, that is cleavable under cellular conditions. For example, Traut's reagent, dithiobis (succinimidylpropionate) (DSP), and N-succinimidyl 3-(2-pyridyldithio)propionate (SPDP) are well-known cleavable cross-linkers. The use of a cleavable cross-linking reagent permits component (II) to separate from component (I) after delivery into the target cell, provided the cell is capable of cleaving a particular sequence of the crosslinker reagent.. For this purpose, direct disulfide linkage may also be useful. Chemical cross-linking may also include the use of spacer arms. Spacer arms provide intramolecular flexibility or adjust intramolecular distances between conjugated moieties and thereby may help preserve biological activity. A spacer arm may be in the form of a protein (or polypeptide) moiety that includes spacer amino acids, e.g. proline. Alternatively, a spacer arm may be part of the cross-linking reagent, such as in "long-chain SPDP" (Pierce Chem. Co., Rockford, III., cat. No. 21651 H). Numerous cross-linking reagents, including the ones discussed above, are commercially available. Detailed instructions for their use are readily available from the commercial suppliers. A general reference on protein cross-linking and conjugate preparation is: Wong, Chemistry of Protein Conjugation and Cross-Linking, CRC Press (1991).

The inventive fusion peptide as defined above optionally may contain an additional component (III). Such a component (III) is preferably a tag for purification of the fusion peptide subsequent to synthesis. A "tag for purification" in the context of the present invention may be of any variety of tags suitable for purification of recombinant proteins such as a hexahistidine-tag (his-tag, polyhistidine-tag), a streptavidine tag (strep-tag), a SBP-tag (a streptavidine binding tag), a GST(glutathione-S-transferase)-tag, a dansyl-tag, etc., or by means of an antibody epitope (an antibody binding tag), e.g. a myc-tag, a Swa11-epitope, a FLAG-tag, etc.. Tags as mentioned above are preferably located at the C-terminus of the (entire) fusion peptide as defined above composed of components (I), (II) and, optionally, component (III). E.g. tags as mentioned above are preferably located at the C-terminus of component (I), if component (I) is located at the C-terminal end of the fusion peptide, or more preferably, at the C-terminus of component (II), if component (II) is located at the C-terminal end of the fusion peptide. Furthermore, a component (III) as mentioned above is preferably selected such as not to interfere with the biological functionality of either component (I) or (II), e.g. the capability of component (I) of the inventive fusion peptide to penetrate the cell membrane, and the capability of component (II) to inhibit interaction of NMDAR and NMDAR interacting proteins. Thus, non-bulky tags are preferably selected as component (III), such as a hexahistidine-tag, a streptavidine tag (strep-tag), a SBP-tag (a streptavidine binding tag), or an antibody epitope, e.g. a myc-tag, a Swa 11-epitope, a FLAG-tag, etc..

Furthermore, component (III) as defined above, if present as an oligo- or polypeptide, may be composed either of D-amino acids as defined above or of naturally occurring amino acids, i.e. L-amino acids. Accordingly, the entire fusion peptide, comprising components (I), (II) and, optionally component (III), as well as linker(s) as defined above, may be composed of D-amino acids as defined above. If the entire inventive fusion peptide, comprising components (I), (II) and, optionally component (III), as well as the linker as defined above, is composed of D-amino acids, the fusion peptide as a whole may be prepared as disclosed above for D-enantiomeric components of the inventive fusion peptide, e.g. using chemical synthesis methods or any other suitable method.

Generally, components (I), (II) and optionally (III) of the inventive fusion peptide as defined above may be arranged suitably, i.e. in any order allowing component (I) to direct the inventive fusion peptide across the plasma membrane, when arranged in the inventive fusion peptide. Furthermore, component (II) still shall be capable to inhibit interaction of N-methyl-D-aspartate receptors and NMDAR interacting proteins. According to a preferred embodiment, component (I) may be located at the N-terminal end of the inventive fusion peptide and component (II) may be located at the C-terminal end of the inventive fusion peptide. Furthermore, if a component (III) as defined above is contained in the inventive fusion peptide, component (III) may be located at the very C-terminal end of the entire inventive fusion peptide. A preferred arrangement of the inventive fusion peptide thus may display from N- to C-terminus component (I), component (II), and, optionally, component (III). However, also other arrangements may be chosen, e.g. from N- to C-terminus component (II), component (I), and, optionally, component (III). If suitable, linkers may be inserted between components (I) and (II) as defined above.

The inventive fusion peptide may also contain a "derivative" of a component (I) and/or a component (II) as defined above. A derivative of a component (I) and/or (II) according to the invention is intended to mean a sequence of a component (I) or (II), which is derived from the naturally occurring (L-amino-acid) sequence of a component (I) or (II) as defined above by way of substitution(s) of one or more amino acids at one or more of sites of the amino acid sequence, by way of deletion(s) of one or more amino acids at any site of the naturally occurring sequence, and/or by way of insertion(s) of one or more amino acids at one or more sites of the naturally occurring peptide sequence. "Derivatives" shall retain their biological activity if used as component (I) or (II) of the inventive fusion peptide, e.g. a derivative of component (I) shall be capable to direct the inventive fusion peptide into the cell cytoplasm or, even further, to a specific cellular destination, as defined above, whereas a derivative of component (I) shall be capable to inhibit interaction of neuronal N-methyl-D-aspartate receptors (NMDARs) and NMDAR interacting proteins. Derivatives in the context of the present invention may also occur in form of their L- or D-amino-acid sequences as defined above, or both.

If substitution(s) of amino acid(s) are carried out for the preparation of a derivative of component (I) and/or component (II) of the inventive fusion peptide, conservative (amino acid) substitutions are preferred. Conservative (amino acid) substitutions typically include substitutions within the following groups: glycine and alanine; valine, isoleucine and leucine; aspartic acid and glutamic acid; asparagine and glutamine; serine and threonine; lysine and arginine; and phenylalanine and tyrosine. Thus, preferred conservative substitution groups are aspartate-glutamate; asparagine-glutamine; valine-leucine-isoleucine; alanine-valine; phenylalanine-tyrosine and lysine-arginine. By such mutations e.g. stability and/or effectiveness of components (I) and (II) of the inventive fusion peptide may be enhanced. If mutations are introduced into components (I) and (II) of the inventive fusion peptide, these components (I) and (II) remain (functionally) homologous, e.g. in sequence, in function, and in antigenic character or other function, with a protein having the corresponding parent sequence. Such mutated components (I) and (II) of the inventive fusion peptide can possess altered properties which may be advantageous over the non-altered sequences of components (I) and (II) for certain applications (e.g. increased pH optimum, increased temperature stability etc.).

A derivative of component (I) or a derivative of component (II), respectively, of the inventive fusion peptide is defined as to have substantial identity with the non-modified sequences of component (I) or component (II), respectively, of the inventive fusion peptide as defined above, e.g. with the HIV TAT protein translocation sequence if used as component (I). Particularly preferred are amino acid sequences which have at least 30% sequence identity, preferably at least 50% sequence identity, even preferably at least 60% sequence identity, even preferably at least 75% sequence identity, even more preferably at least 80%, yet more preferably 90% sequence identity and most preferably at least 95% or even 99% sequence identity to the naturally occurring analogue. Appropriate methods for synthesis or isolation of a functional derivative of components (I) and (II) of the inventive fusion peptide as well as for determination of percent identity of two amino acid sequences are described above. Additionally, methods for production of derivatives of components (I) and (II) of the inventive fusion peptide as disclosed above are well known and can be carried out following standard methods which are well known by a person skilled in the art (see e.g., Sambrook J, Maniatis T (1989) *supra*).

As a further embodiment, the invention provides pharmaceutical compositions comprising the inventive fusion peptide as defined above. Preferably, such pharmaceutical compositions comprise the inventive fusion peptide with components (I) and (II) and, optionally, component (III) as well as an optional linker, as defined above. Additionally, such a pharmaceutical composition may comprise a pharmaceutically acceptable carrier, adjuvant, or vehicle. A "pharmaceutically acceptable carrier, adjuvant, or vehicle" according to the invention refers to a non-toxic carrier, adjuvant or vehicle that does not destroy the pharmacological activity of the inventive fusion peptide with which it is formulated. Pharmaceutically acceptable carriers, adjuvants or vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of the present invention may be administered orally, parenterally, by inhalation spray, topically, rectally, nasally, buccally, vaginally or via an implanted reservoir.

The term parenteral as used herein includes subcutaneous, intravenous, intramuscular, intra-articular, intra-synovial, intrasternal, intrathecal, intrahepatic, intralesional and intracranial injection or infusion techniques. Preferably, the pharmaceutical compositions are administered orally, intraperitoneally or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutically acceptable compositions herein may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavouring or colouring agents may also be added.

Alternatively, the inventive pharmaceutical composition as defined herein may be administered in the form of suppository for rectal administration. Such a suppository can be prepared by mixing the agent with a suitable non-irritating excipient that is solid at room temperature but liquid at rectal temperature and, therefore, will melt in the rectum to release the drug. Such materials include cocoa butter, beeswax and polyethylene glycols.

The inventive pharmaceutical composition as defined herein may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the inventive pharmaceutical composition as defined herein may be formulated in a suitable ointment containing the inventive fusion peptide as identified above suspended or dissolved in one or more carriers. Carriers for topical administration of the inventive fusion peptide include, but are not limited to, mineral oil, liquid petrolatum, white petrolatum, propylene glycol, polyoxyethylene, polyoxypropylene compound, emulsifying wax and water. Alternatively, the inventive pharmaceutical composition as defined herein can be formulated in a suitable lotion or cream containing the inventive fusion peptide suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the inventive pharmaceutical composition may be formulated as micronized suspensions in isotonic, pH adjusted sterile saline, or, preferably, as a solution in isotonic, pH adjusted sterile saline, either with or without a preservative such as benzylalkonium chloride. Alternatively, for ophthalmic uses, the pharmaceutically acceptable composition may be formulated in an ointment such as petrolatum.

The inventive pharmaceutical composition as defined herein may also be administered by nasal aerosol or inhalation. Such a composition may be prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutically acceptable composition herein is formulated for oral or parenteral administration, e.g. by injection.

For treatment purposes, a non-toxic, damage-reducing, effective amount of the inventive fusion peptide may be used for preparation of an inventive pharmaceutical composition as defined above. Therefore, an amount of the inventive fusion peptide may be combined with the carrier material(s) to produce a composition as defined above. The inventive pharmaceutical composition is typically prepared in a single (or multiple) dosage form, which will vary depending upon the host treated and the particular mode of administration. Usually, the inventive pharmaceutical composition is formulated so that a dosage range per dose of between 0.001-100 mg/kg body weight/day of the fusion peptide can be administered to a patient receiving the inventive pharmaceutical composition. Preferred dosage ranges per dose vary from 0.01 - 50 mg/kg body weight/day, even further preferred dosage ranges per dose vary from 0.1 - 25 mg/kg body weight/day. However, dosage ranges and treatment regimens as mentioned above may be adapted suitably for any particular patient dependent upon a variety of factors, including the activity of the specific inventive fusion peptide employed, the age, body weight, general health, sex, diet, time of administration, rate of excretion, drug combination, the judgment of the treating physician and the severity of the particular disease being treated. In this context, administration may be carried with in an initial dosage range, which may be varied over the time of treatment, e.g. by increasing or decreasing the initial dosage range within the range as set forth above. Alternatively, administration may be carried out in a continuous manner by administering a specific dosage range, thereby maintaining the initial dosage range over the entire time of treatment. Both administration forms may furthermore be combined, e.g. if the dosage range is to be adapted (increased or decreased) between various sessions of the treatment but kept constant within the single session so that dosage ranges of the various sessions differ from each other.

The inventive pharmaceutical composition may be employed for treatment of cerebral stroke, ischemic cerebral stroke as well as of anoxia, ischemia, neuropathic pain, particularly neuropathic pain related to PSD-95 and/or NMDAR interaction, etc.. Furthermore, the inventive pharmaceutical composition may be employed for providing neuroprotective effect against or treatment of, chronic neurodegenerative conditions Excitotoxicity may be particularly involved in stroke traumatic brain injury and neurodegenerative diseases of the central nervous system (CNS) such as Multiple sclerosis, Alzheimer's disease, Amyotrophic lateral sclerosis (ALS), Fibromyalgia, Parkinson's disease, and Huntington's disease. Other common conditions that cause excessive glutamate concentrations around neurons are hypoglycemia and status epilepticus, glaucoma/deterioration of retinal ganglion cells, etc..

The treatment of diseases or disorders as mentioned herein is typically carried out by administering an inventive pharmaceutical composition as defined about in a dosage range as defined above. Administration of the inventive pharmaceutical composition may be carried out either prior to onset of excitotoxiticity and/or (ischemic) brain damage, i.e. the damaging effect of an injury to mammalian cells, or concurrent or subsequent thereto; E.g. administration of the inventive pharmaceutical composition may be carried out within a time of (up to) 1 hour (0-1 hours), up to 2 hours, up to 3-5 hours or up to 24 hours or more subsequent to a cerebral stroke or spinal cord injuries, ischemic or traumatic injuries to the brain or spinal cord and, in general, damages to the central nervous system (CNS) neurons.

The present invention furthermore provides kits comprising the above defined inventive pharmaceutical composition (in one or more container(s)) in at least one of the above formulations and an instruction manual or information brochure regarding instructions and/or information with respect to application of the inventive pharmaceutical composition.

Although this disclosure has described and illustrated certain preferred embodiments of the invention, it is to be understood that the invention is not restricted to those particular embodiments.

### Description of Figures

The following Figures are intended to further illustrate the present invention without limiting the scope of the invention thereto.

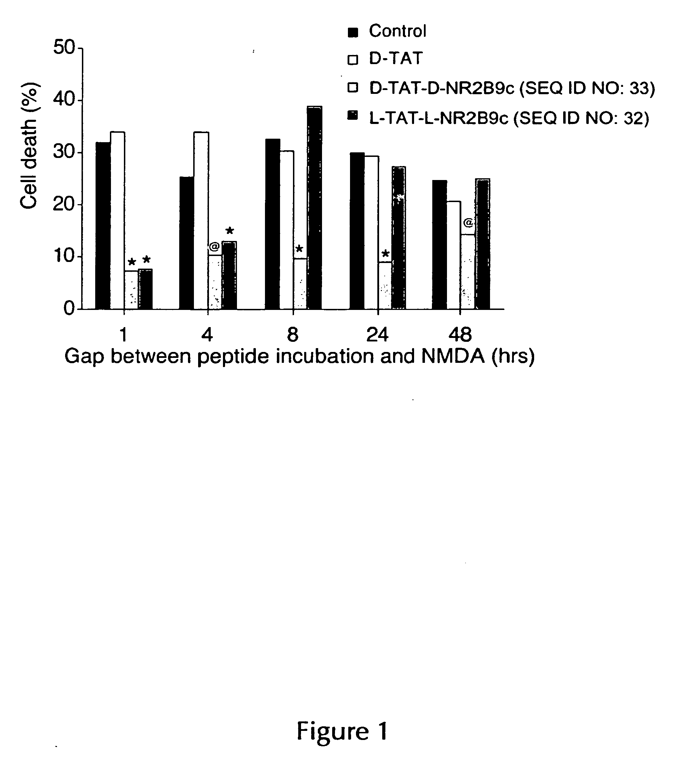

- Figure 1:: shows the duration of efficacy of fusion peptides according to SEQ ID NOs: 32 and 33 in the presence of 20 µM NMDA, particularly TAT-NR2B9c peptides in L-form (L-TAT-L-NR2B9c sequence YGRKKRRQRRR-KLSSIESDV (SEQ ID NO: 32), identical to US 20030050243) versus their D-form (D-TAT- D-NR2B9c, sequence vdseisslk-rrrqrrkkrgy (SEQ ID NO: 33)). In this experiment (as well as in the following experiments), the cell death rate was measured parallel to addition of peptides capable of inhibiting cell death by interacting with the NMDA receptor. As can be seen in Fig. 1, in any single experiment D-TAT-D-NR2B9c according to SEQ ID NO: 33 shows at least equal, but predominantly significantly increased efficacy versus the comparative compound L-TAT-L-NR2B9c according to SEQ ID NO: 32, i.e. significantly lowered cell death rates (%) of neuronal cells; * in Fig. 1 indicates P<0.05, @ P<0.07 (Paired t-test, comparing peptide-treated neurons with control neurons). In particular, D-TAT-D-NR2B9c discloses its positive effect, if the gap between peptide incubation and NMDA addition is ≥ 8 h. This reveals the prolonged pharmacological activity of the D-Form. The most efficient therapy window was observed between 4 and 48 hrs, particularly 8 and 24 hrs.

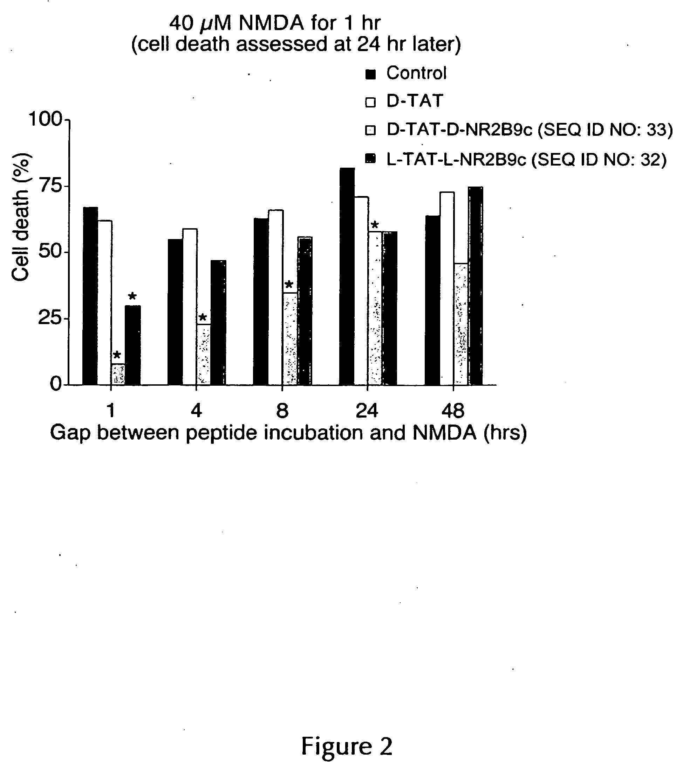

- Figure 2:: shows the duration of efficacy of fusion peptides according to SEQ ID NOs: 32 and 33 in the presence of 40 µM NMDA, particularly TAT-NR2B9c peptides in L-form (L-TAT-L-NR2B9c sequence YGRKKRRQRRR-KLSSIESDV (SEQ ID NO: 32), identical to US 20030050243) versus its D-form (D-TAT- D-NR2B9c, sequence vdseisslk-rrrqrrkkrgyin (SEQ ID NO: 33)). As can be seen in Fig. 2, in any single experiment D-TAT-D-NR2B9c according to SEQ ID NO: 33 shows at least equal, but predominantly significantly increased efficacy versus the comparative compound L-TAT-L-NR2B9c according to SEQ ID NO: 32, i.e. significantly lowered cell death rates (%) of neuronal cells. * in Fig. 2 indicates P < 0.05, @ P<0.07 (paired t-test, comparing peptide-treated neurons with control neurons). The remarkable positive effect of the inventive fusion peptide becomes apparent, if the time window is less than 24 h.

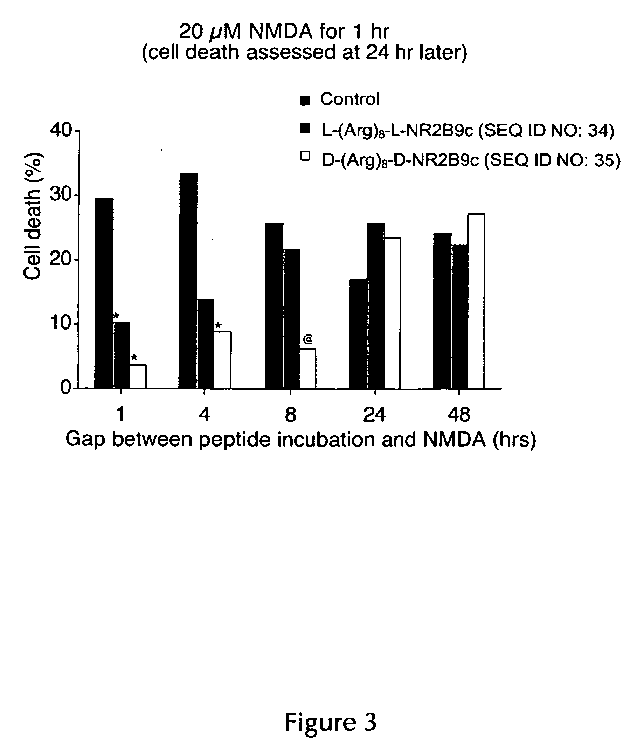

- Figure 3:: shows the duration of efficacy of fusion peptides according to SEQ ID NOs: 34 and 35 the presence of 20 µM NMDA, particularly (Arg)₈-NR2B9c peptides in L-form (L-(Arg)₈-L-NR2B9c sequence RRRRRRRR-KLSSIESDV (SEQ ID NO: 34)) versus its D-form (D-(Arg)₈-D-NR2B9c, sequence vdseisslk- rrrrrrrr (SEQ ID NO: 35)). As can be seen in Fig. 3, in any single experiment inventive fusion peptide D-(Arg)₈-D-NR2B9c according to SEQ ID NO: 35 shows at least equal, but predominantly significantly increased efficacy versus the comparative compound L-(Arg)₈-L-NR2B9c according to SEQ ID NO: 34, i.e. significantly lowered cell death rates (%) of neuronal cells; * in Fig. 3 indicates P < 0.05, @ P<0.07 (paired t-test, comparing peptide-treated neurons with control neurons).

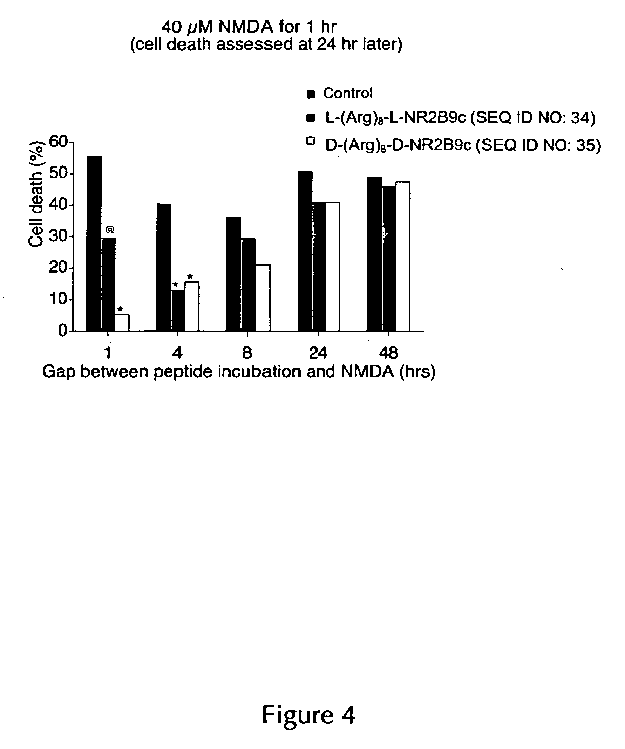

- Figure 4:: shows the duration of efficacy of fusion peptides according to SEQ ID NOs: 34 and 35 the presence of 40 µM NMDA, particularly (Arg)₈-NR2B9c peptides in L-form (L-(Arg)₈-L-NR2B9c sequence RRRRRRRR-KLSSIESDV (SEQ ID NO: 34)) versus its D-form (D-(Arg)₈-D-NR2B9c, sequence vdseisslk- rrrrrrrr (SEQ ID NO: 35)). As can be seen in Fig. 4, in any single experiment up to the 8 hr time point inventive fusion peptide D-(Arg)₈-D-NR2B9c according to SEQ ID NO: 35 shows at least equal, but predominantly significant increased efficacies versus the comparative compound L-(Arg)₈-L- NR2B9c according to SEQ ID NO: 34, i.e. significantly lowered cell death rates (%) of neuronal cells; * in Fig. 4 indicates P < 0.05, @ P<0.07 (paired t- test, comparing peptide-treated neurons with control neurons).

### Examples

The following Examples are intended to further illustrate the present invention without limiting the scope of the invention to these examples.

### Example 1 Synthesis of inventive fusion peptides

TAT-NR2B9C peptides were synthesized manually on MBHA resin (Novabiochem, Merck) by N^{α}-Boc chemistry SPPS, according to standard procedures. All couplings were monitored by the TNBSA color test. The peptides were cleaved and simultaneously deprotected from the resin by treatment with 90% HF with appropriate scavengers 1hr at 0 °C . The crude peptides were taken up in a 20% aqueous acetic acid solution, diluted to a 5% acetic acid solution with doubly distilled water and remaining scavengers were extracted by a diethylether wash. The solutions containing the peptides were then lyophilized to remove the acetic acid. Peptides UD-NR2B9C were purified by preparative RP-HPLC on a Atlantis (Waters) dC₁₈ column (15 - 45% buffer B over 45 min at 15 mL/min) and characterized by ESI-MS.

(Arg)₈-NR2B9C peptides were synthesized manually, The synthesis was carried out on solid support using a Fmoc strategy on a Fmoc-Val-NovaSyn TGA resin from Novabiochem, with a loading of 0.22 mmol/g. The synthesis was done on a ACT robot, in a well corresponding to 145 µmol, using 4 equivalents of amino acids, 4 equivalents of coupling agents HOBt and 4 equivalents of DIPCDI. Due to synthetic requirements double coupling was done every 3 amino acids:
- the first coupling with 4 equivalents of amino acids, 4 equivalents of HOBt and 4 equivalents of DIPCDI
- the second coupling with 4 equivalents of amino acids, 4 equivalents HATU and 4 equivalents of DIEA

After each coupling, a capping step was carried out with acetic anhydride 10% in DMF (for 15 minutes).

Deprotection steps were carried out with 20% piperidine in DMF (2x20 minutes). The Fmoc in position "n" was kept until the end of the synthesis. Cleavage was carried out in a 86% TFA solution in presence of scavengers. The peptide was precipitated in ether. Then, the peptide was purified on a C-1.8 reverse phase column using a gradient of 0 to 60% acetonitrile in 60 minutes, with UV-detection at 220 nm (for peptidic bonds) and at 300 nm (for Fmoc protection). Subsequently, the peptide was lyophilized with the Fmoc protecting group, which was removed afterwards by treatment with a 20 equivalents solution of DEA. The peptide was then purified again (using the same conditions as described above) before being lyophilized, analyzed and used in subsequent experiments.

### Example 2 Test of duration of neuroprotection afforded by TAT-NR2B9c in the face of excitotoxic insults

### Protocol:

Rat cortical neurons, taken from E21 rats, were cultured for 7-9 days *in vitro* in Neurobasal-A medium + B-27 supplement (both Invitrogen), 1 mM glutamine, + 50 units/ml penicillin, 50µg/m) streptomycin.
1. At varying times prior to compound exposure, neurons were removed from Neurobasal-A based culture medium, and placed into "transfection medium" (TM; Bading et al. (1993), Science 260, 181-186): 10% (Minimum Essential Medium, Invitrogen (21090022), containing Earles salt, but no L-glutamine), 90% Salt-Glucose-Glycine (SGG) medium SGG: 114 mM NaCl, 0.219 % NaHCO₃, 5.292 mM KCI, 1 mM MgCl₂, 2 mM CaCl₂, 10 mM HEPES, 1 mM Glycine, 30 mM Glucose, 0.5 mM sodium pyruvate, 0.1 % Phenol Red, insulintransferrin-selenite supplement (Sigma, 7.5 µg insulin/ml; 7.5 µg transferrin/ml and 7.5 ng sodium selenite/ml); final osmolarity 325mosm/l). All subsequent steps took place in TM.
2. Neurons were then exposed to inventive fusion peptides at 10 µM for 1 hr. (particularly TAT-NR2B9c peptides in L-form (L-TAT-L-NR2B9c sequence YGRKKRRQRRR-KLSSIESDV (SEQ ID NO: 32), identical to US 20030050243) and their D-form (D-TAT-D-NR2B9c, sequence vdseisslk-rrrqrrkkrgyin (SEQ ID NO: 33)))
3. After 1 hr neurons were washed once in TM to remove peptide.
4. After a varying period of time, neurons were exposed to NMDA at either 20 or 40 µM for 1 hr, after which neurons are placed in TM for 24 h.
5. Neurons were fixed (3 % paraformaldehyde) and stained (DAPI, 4',6-Diamidino-2-phenylindole). Number of pyknotic nuclei as a % of total is counted. Experiments were performed in triplicate.

### Results

Both D- and L- forms of TAT-NR2B9c (L-TAT-L-NR2B9c sequence YGRKKRRQRRR-KLSSIESDV (SEQ ID NO: 32), identical to US 20030050243), D-TAT-D-NR2B9c, sequence vdseisslk-rrrqrrkkrgyin (SEQ ID NO: 33)) offered protection for up to 4 hrs after washout of the peptide in the face of NMDA (20 and 40 µM). However, protection by the D-form extended to up to 48 h post-washout for the lower dose of NMDA (Fig. 1), indicating that it is a more promising candidate as a therapeutic drug. It also provided significant protection for up to 24 hours in the face of 40 µM NMDA, while neuroprotection obtained with the L-form was only significant at 1 hour (Fig. 2).

### Example 3 Test of duration of neuroprotection afforded by (Arg)₈-NR2B9c in the face of excitotoxic insults

### Protocol:

Rat cortical neurons, taken from E21 rats, were cultured for 7-9 days *in* vitro in Neurobasal-A medium + B-27 supplement (both Invitrogen), 1 mM glutamine, + 50 units/ml penicillin, 50µg/ml streptomycin
1. At varying times prior to peptide exposure, neurons were removed from Neurobasal-A based culture medium, and placed into TM (see above) with the inclusion of Insulintransferrin-selenite supplement (Sigma). All subsequent steps took place in TM (see above).
2. Neurons were then exposed to inventive fusion peptides at 10 µM for 1 hr. (particularly (Arg)₈-NR2B9c peptides in D-form (D-TAT-D-NR2B9c, sequence vdseisslk-rrrrrrrr (SEQ ID NO: 35)) and also L-form (SEQ ID NO: 34))
3. After 1 hr neurons were washed once in TM to remove peptide.
4. After a varying period of time, neurons were exposed to NMDA for 1 hr, after which neurons are placed in TM for 24 h.
5. Neurons were fixed (3 % paraformaldehyde) and stained (DAPI 4',6-Diamidino-2-phenylindole). The number of pyknotic nuclei as a % of total was counted. Experiments were performed in triplicate.

### Results

The D-form of (Arg)₈-NR2B9c (D-(Arg)₈-D-NR2B9c (SEQ ID NO: 35) offered superior neuroprotection for up to 8 hours after washout of the peptide in the face of NMDA (20 µM) versus the D-form (Fig. 3). At the higher NMDA dose (40 µM), the D-form also showed equal to better protective effects (Fig. 4).. These results suggest that the D-form, is a more promising candidate as a therapeutic drug. Overall, D-(Arg)₈-D-NR2B9c (SEQ ID NO: 35)) performs similar to D-TAT-D-NR2B9c (SEQ ID NO: 33)), albeit with a shorter duration of neuroprotective activity, which may suggest a more pronounced cell-penetrating capacity of D-TAT than D-(Arg)₈.

## Claims

1. A fusion peptide comprising at least a component (I), wherein component (I) comprises a cell membrane penetrating peptide, and a component (II), selected from a peptide comprising the sequence vdseisslk (SEQ. ID NO: 31) or a peptide comprising a sequence with a sequence identity of about 60, 70, 80, 90, 95 or even 99 % with the sequence of SEQ ID NO: 31, wherein component (II) entirely consists of D-enantiomeric amino acids, wherein component (I) directs the fusion peptide across the cell membrane and wherein component (II) is in the fusion peptide still capable to inhibit interaction of N-methyl-D-aspartate receptor (NMDAR) with NMDAR interacting proteins.

2. Fusion peptide according to claim 1, wherein component (I) is selected from cell penetrating peptides including:
(a) protein transduction domains (PTD) and protein derived CPPs, including sequences derived from Antennapedia, pAntp (43-58), comprising the sequences RQIKIWFQNRRMKWKK (SEQ ID NO: 1) or RQIKIWFQNRRMKWKK-amide (SEQ ID NO: 2), including sequences derived from human immunodeficiency virus 1 (HIV-1), TAT, region 37 to 72, region 37 to 60, region 48 to 60 or region 49 to 57 from TAT, sequences GRKKRRQRRR (SEQ ID NO: 3), YGRKKRRQRRR (SEQ ID NO: 4), CGRKKRRQRRRPPQC (SEQ ID NO: 5) or CGRKKRRQRRRPPQCC (SEQ ID NO: 6), including hCT(9-32) having the sequence LGTYTQDFNKFHTFPQTAIGVGAP-NH₂ (SEQ ID NO: 7), pVEC comprising the sequence LLIILRRRIRKQAHAHSK-NH₂ (SEQ ID NO: 8), pISL comprising the sequence RVIRVWFQNKRCKDKK-NH₂(SEQ ID NO: 9), mouse PRP (1-28) comprising the sequence MANGLYWLLALFVTMWTDVGLCKKRPKP-NH₂ (SEQ ID NO: 10) and homologs thereto including human homologs, E^{ms} (194-220) comprising the sequence RQGAARVTSWLGLQLRIAGKRLEGRSK-NH₂ (SEQ ID NO: 11), Restricocin L3 (60-73) comprising the sequence KLIKGRTPIKFGK-NH₂ (SEQ ID NO: 12), or
(b) model peptides including VT5 comprising the sequence DPKGDPKGVTVTVTVTVTGKGDPKPD-NH₂ (SEQ ID NO: 13), MAP comprising the sequence KLALKLALKALKAALKLA-NH₂ (SEQ ID NO: 14), arginine stretches including RRRRRRR, i.e. (Arg)₇ (SEQ ID NO: 15), or RRRRRRR-NH₂, i.e. (Arg)₇-NH₂ (SEQ ID NO: 16), or RRRRRRR-C, i.e. (Arg)₇-C (SEQ ID NO: 17), or RRRRRRRR, i.e. (Arg)₈ (SEQ ID NO: 18), or RRRRRRRR-NH₂, i.e. (Arg)₈-NH₂ (SEQ ID NO: 19), RRRRRRRRR, i.e. (Arg)₉ (SEQ ID NO: 20), or RRRRRRRRR-NH₂, i.e. (Arg)₉-NH₂, (SEQ ID NO: 21), or
(c) designed CPPs including MPG comprising the sequence GALFLGFLGAAGSTMGAWSQPKSKRKV (SEQ ID NO: 22) or GALFLGFLGAAGSTMGAWSQPKSKRKV-cysteamide (SEQ ID NO: 23), Transportan comprising the sequence GWTLNSAGYLLGKINLKALAALAKKIL-NH₂ (SEQ ID NO: 24), Transportan 10 comprising the sequence AGYLLGKINLKALAALAKKIL-NH₂ (SEQ ID NO: 25), Pep-1 comprising the sequence KETWWETWWTEWSQPKKKRKV (SEQ ID NO: 26) or KETWWETWWTEWSQPKKKRKV-cysteamide (SEQ ID NO: 27),
or may be selected from the KALA peptide comprising the sequence WEAKLAKALAKALAKHLAKALAKALKACEA (SEQ ID NO: 28) or from Bulforin 2 comprising the sequence TRSSRAGLQFPVGRVHRLLRK (SEQ ID NO: 29),
or an retro-inverso isomer of SEQ ID Nos: 1-29 composed of D amino acids.

3. Fusion peptide according to claim 2, wherein component (I) is selected from cell penetrating peptides showing a sequence identity of about 60, 70, 80, 90, 95 or even 99 % with a sequence according to any of SEQ ID NOs: 1 to 29 as defined in claim 2, provided that component (I) still retains its biological activity, i.e. to direct the inventive fusion peptide across the plasma membrane.

4. Fusion peptide according to any of claims 1 to 3, wherein component (I) entirely consists of L-amino acids, D-amino acids, or both.

5. Fusion peptide according to any of claims 1 to 4, wherein components (I) and (II) are linked by a linker.

6. Fusion peptide according to any of claims 1 to 5, additionally comprising a component (III), wherein component (III) is a tag for purification.

7. Fusion peptide according to any of claims 1 to 6 for use as medicament.

8. Pharmaceutical composition comprising a fusion peptide according to any of claims 1 to 6 and optionally a pharmaceutical carrier.

9. Use of a fusion peptide according to any of claims 1 to 6 for preparing a pharmaceutical composition for treatment of diseases selected from cerebral stroke, ischemic cerebral stroke, anoxia, ischemia, chronic neurodegenerative conditions and neuropathic pain.

10. Kit comprising a pharmaceutical composition according to claim 8 and an instruction manual or information brochure with instructions and/or information for application of the pharmaceutical composition.

11. Fusion peptide according to any of claims 1 to 6 for use in a method of treatment of diseases selected from cerebral stroke, ischemic cerebral stroke, anoxia, ischemia, chronic neurodegenerative conditions and neuropathic pain.

## Patentansprüche

1. Fusionspeptid, umfassend mindestens einen Bestandteil (I), wobei Bestandteil (I), ein Zellmembran durchdringendes Peptid umfasst, und einen Bestandteil (II), ausgewählt aus einem Peptid, umfassend die Sequenz vdseisslk (SEQ ID NO: 31) oder einem Peptid, das eine Sequenz mit einer Sequenzidentität von etwa 60, 70, 80, 90, 95 oder sogar 99% mit der Sequenz von SEQ ID NO: 31 umfasst, wobei Bestandteil (II) vollständig aus D-enantiomeren Aminosäuren besteht, wobei Bestandteil (I) das Fusionspeptid durch die Zellmembran führt und wobei Bestandteil (II) in dem Fusionspeptid noch fähig ist, eine Wechselwirkung des N-Methyl-D-Aspartat Rezeptors (NMDAR) mit NMDAR-wechselwirkenden Proteinen zu hemmen.

2. Fusionspeptid gemäß Anspruch 1, wobei Bestandteil (I) aus zelldurchdringenden Peptiden ausgewählt ist, einschließlich:
(a) Proteintransduktionsdomänen (PTD) und proteinabgeleitete CPPs, einschließlich Sequenzen abgeleitet von Antennapedia, pAntp (43-58), umfassend die Sequenzen RQIKIWFQNRRMKWKK (SEQ ID NO: 1) oder RQIKIWFQNRRMKWKK-Amid (SEQ ID NO: 2), einschließlich Sequenzen abgeleitet von dem humanen Immundefizienz Virus 1 (HIV-1), TAT, Bereich 37 bis 72, Bereich 37 bis 60, Bereich 48 bis 60 oder Bereich 49 bis 57 von TAT, Sequenzen GRKKRRQRRR (SEQ ID NO: 3), YGRKKRRQRRR (SEQ ID NO: 4), CGRKKRRQRRRPPQC (SEQ ID NO: 5) oder CGRKKRRQRRRPPQCC (SEQ ID NO: 6), einschließlich hCT(9-32) mit der Sequenz LGTYTQDFNKFHTFPQTAIGVGAP-NH₂ (SEQ ID NO: 7), pVEC umfassend die Sequenz LLIILRRRIRKQAHAHSK-NH₂ (SEQ ID NO: 8), pISL umfassend die Sequenz RVIRVWFQNKRCKDKK-NH₂ (SEQ ID NO: 9), Maus PRP (1 - 28) umfassend die Sequenz MANGLYWLLALFVTMWTDVGLCKKRPKP-NH₂ (SEQ ID NO: 10) und Homologe dazu einschließlich menschliche Homologe, E^{ms} (194-220) umfassend die Sequenz RQGAARVTSWLGLQLRI-AGKRLEGRSK-NH₂ (SEQ ID NO: 11), Restricocin L3 (60-73) umfassend die Sequenz KLIKGRTPIKFGK-NH₂ (SEQ ID NO: 12), oder
(b) Modelpeptide, einschließlich VT5 umfassend die Sequenz DPKGDPKGVTVTVTVTVTGKGDPKPD-NH₂ (SEQ ID NO: 13), MAP umfassend die Sequenz KLALKLALKALKAALKLA-NH₂ (SEQ ID NO: 14), Argininabschnitte einschließlich RRRRRRR, d.h. (Arg)₇ (SEQ ID NO: 15), oder RRRRRRR-NH₂, d.h. (Arg)₇-NH₂ (SEQ ID NO: 16), oder RRRRRRR-C, d.h. (Arg)₇-C (SEQ ID NO: 17), oder RRRRRRRR, d.h. (Arg)₈ (SEQ. ID NO: 18), oder RRRRRRRR-NH₂, d.h. (Arg)₈-NH₂ (SEQ ID NO: 19) RRRRRRRRR, d.h. (Arg)₉ (SEQ ID NO: 20), oder RRRRRRRRR-NH₂, d.h. (Arg)₉-NH₂, (SEQ ID NO: 21), oder
(c) entworfene CPPs einschließlich MPG umfassend die Sequenz GALFLGFLGAAGSTMGAWSQPKSKRKV (SEQ ID NO: 22) oder GALFLGFLGAAGSTMGAWSQPKSKRKV-Cysteamid (SEQ ID NO: 23), Transportan umfassend die Sequenz GWTLNSAGYLLGKINLKALAALAKKIL-NH₂ (SEQ ID NO: 24), Transportan 10 umfassend die Sequenz AGYLLG-KINLKALAALAKKIL-NH₂ (SEQ ID NO: 25), Pep-1 umfassend die Sequenz KETWWETWWTEWSQPKKKRKV (SEQ ID NO: 26) oder KETWWETWWTEWSQPKKKRKV-Cysteamid (SEQ ID NO: 27),
oder ausgewählt sein kann aus dem KALA Peptid umfassend die Sequenz WEAKLAKALAKALAKHLAKALAKALKACEA (SEQ ID NO: 28) oder aus Bulforin 2 umfassend die Sequenz TRSSRAGLQFPVGRVHRLLRK (SEQ ID NO: 29),
oder einem retro-inverso Isomer der SEQ ID Nos: 1-29 welches aus D-Aminosäuren gebildet ist.

3. Fusionspeptid gemäß Anspruch 2, wobei Bestandteil (I) aus zelldurchdringenden Peptiden ausgewählt ist, die eine Sequenzidentität von etwa 60, 70, 80, 90, 95 oder sogar 99 % mit einer Sequenz gemäß einer der SEQ ID NOs: 1 to 29 wie in Anspruch 2 definiert zeigt, vorausgesetzt, dass Bestandteil (1) seine biologische Aktivität noch behält, d.h. das erfinderische Fusionspeptid durch die Plasmamembran zu führen.

4. Fusionspeptid gemäß einem der Ansprüche 1 bis 3, wobei Bestandteil (I) vollständig aus L-Aminosäuren, D-Aminosäuren oder beiden besteht.

5. Fusionspeptid gemäß einem der Ansprüche 1 bis 4, wobei Bestandteile (I) und (II) über einen Linker verbunden sind.

6. Fusionspeptid gemäß einem der Ansprüche 1 bis 5, zusätzlich einen Bestandteil (III) umfassend, wobei Bestandteil (III) eine Markierung für die Aufreinigung ist.

7. Fusionspeptid gemäß einem der Ansprüche 1 bis 6 für die Verwendung als Medikament.

8. Pharmazeutische Zusammensetzung, die ein Fusionspeptid gemäß einem der Ansprüche 1 bis 6 und gegebenenfalls einen pharmazeutischen Träger umfasst.

9. Verwendung eines Fusionspeptids gemäß einem der Ansprüche 1 bis 6 für die Herstellung einer pharmazeutischen Zusammensetzung für die Behandlung von Erkrankungen, ausgewählt aus Schlaganfall, ischämischem Schlaganfall, Anoxie, Ischämie, chronischen neuro-degenerativen Zuständen und neuropathischem Schmerz.

10. Kit, das eine pharmazeutische Zusammensetzung gemäß Anspruch 8 und ein Anweisungshandbuch oder Informationsbroschüre mit Anweisungen und/oder Information für die Anwendung der pharmazeutischen Zusammensetzung umfasst.

11. Fusionspeptid gemäß einem der Ansprüche 1 bis 6 für die Verwendung in einem Behandlungsverfahren von Erkrankungen, ausgewählt aus Schlaganfall, ischämischem Schlaganfall, Anoxie, Ischämie, chronischen neuro-degenerativen Zuständen und neuropathischem Schmerz.

## Revendications

1. Peptide de fusion comprenant au moins un composant (I), dans lequel le composant (I) comprend un peptide pénétrant la membrane cellulaire, et un composant (II), choisi parmi un peptide comprenant la séquence vdseisslk (SEQ ID NO : 31) et un peptide comprenant une séquence ayant une identité de séquence d'environ 60, 70, 80, 90, 95 ou même 99 % avec la séquence de la SEQ ID NO : 31, dans lequel le composant (II) est entièrement constitué d'acides aminés D-énantiomères, dans lequel le composant (I) dirige le peptide de fusion à travers la membrane cellulaire et dans lequel le composant (II) est, dans le peptide de fusion, toujours capable d'inhiber l'interaction du récepteur de N-méthyl-D-aspartate (NMDAR) avec des protéines interagissant avec le NMDAR.

2. Peptide de fusion selon la revendication 1, dans lequel le composant (I) est choisi parmi les peptides pénétrant les cellules comprenant :
(a) les domaines de transduction de protéine (PTD) et les CPP dérivés de protéine, contenant des séquences dérivées d'Antennapedia, pAntp (43-58), comprenant les séquences RQIKIWFQNRRMKWKK (SEQ ID NO : 1) ou RQIKIWFQNRRMKWKK-amide (SEQ ID NO : 2), contenant des séquences dérivées du virus 1 de l'immunodéficience humaine (VIH-1), TAT, région 37 à 72, région 37 à 60, région 48 à 60 ou région 49 à 57 de TAT, les séquences GRKKRRQRRR (SEQ ID NO : 3), YGRKKRRQRRR (SEQ ID NO : 4), CGRKKRRQRRRPPQC (SEQ ID NO : 5) ou CGRKKRRQRRRPPQCC (SEQ ID NO : 6), contenant hCT(9-32) ayant la séquence LGTYTQDFNKFHTFPQTAIGVGAP-NH₂ (SEQ ID NO : 7), pVEC comprenant la séquence LLIILRRRIRKQAHAHSK-NH₂ (SEQ ID NO : 8), pISL comprenant la séquence RVIRVWFQNKRCKDKK-NH₂ (SEQ ID NO : 9), PRP de souris (1-28) comprenant la séquence MANGLYWLLALFVTMWTDVGLCKKRPKP-NH₂ (SEQ ID NO : 10) et ses homologues y compris les homologues humains, E^{ms} (194-220) comprenant la séquence RQGAARVTSWLGLQLRIAGKRLEGRSK-NH₂ (SEQ ID NO : 11), restricocine L3 (60-73) comprenant la séquence KLIKGRTIPKFGK-NH₂ (SEQ ID NO : 12), ou
(b) les peptides modèles contenant VT5 comprenant la séquence DPKGDPKGVTVTVTVTVTGKGDPKPD-NH₂ (SEQ ID NO : 13), MAP comprenant la séquence KLALKLALKALKAALKLA-NH₂ (SEQ ID NO : 14), les étirements d'arginine contenant RRRRRRR, c'est-à-dire (Arg)₇ (SEQ ID NO : 15), ou RRRRRRR-NH₂, c'est-à-dire (Arg)₇-NH₂ (SEQ ID NO : 16), ou RRRRRRR-C, c'est-à-dire (Arg)₇-C (SEQ ID NO : 17), ou RRRRRRRR, c'est-à-dire (Arg)₈ (SEQ ID NO : 18), ou NH₂, c'est-à-dire (Arg)₈-NH₂ (SEQ ID NO : 19), RRRRRRRRR, c'est-à-dire (Arg)₉ (SEQ ID NO : 20) ou RRRRRRRRR-NH₂, c'est-à-dire (Arg)₉-NH₂ (SEQ ID NO : 21), ou
(c) les CPP spécifiquement conçus contenant MPG comprenant la séquence GALFLGFLGAAGSTMGAWSQPKSKRKV (SEQ ID NO : 22) ou GALFLGFLGAAGSTMGAWSQPKSKRKV-cystéamide (SEQ ID NO : 23), Transportan comprenant la séquence GWTLNSAGYLLGKINLKALAALAKKIL-NH₂ (SEQ ID NO : 24), Transportan 10 comprenant la séquence AGYLLGKINLKALAALAKKIL-NH₂ (SEQ ID NO : 25), Pep-1 comprenant la séquence KETWWETWWTEWSQPKKKRKV (SEQ ID NO : 26) ou KETWWETWWTEWSQPKKKRKV-cystéamide (SEQ ID NO : 27),
ou peut être choisi parmi le peptide KALA comprenant la séquence WEAKLAKALAKALAKHLAKALAKALKACEA (SEQ ID NO : 28) ou Bulforin 2 comprenant la séquence TRSSRAGLQFPVGRVHRLLRK (SEQ ID NO : 29),
ou un isomère rétro-inverse des SEQ ID NO : 1 à 29 composé d'acide D-aminés.

3. Peptide de fusion selon la revendication 2, dans lequel le composant (I) est choisi parmi les peptides pénétrant les cellules présentant une identité de séquence d'environ 60, 70, 80, 90, 95 ou même 99 % avec une séquence selon l'une quelconque des SEQ ID NO : 1 à 29 telles que définies dans la revendication 2, sous réserve que le composant (I) conserve toujours son activité biologique, c'est-à-dire puisse diriger le peptide de fusion de l'invention à travers la membrane plasmatique.

4. Peptide de fusion selon l'une quelconque des revendications 1 à 3, dans lequel le composant (I) est entièrement constitué d'acides L-aminés, d'acides D-aminés, ou des deux.

5. Peptide de fusion selon l'une quelconque des revendications 1 à 4, dans lequel les composants (I) et (II) sont liés par un lieur.

6. Peptide de fusion selon l'une quelconque des revendications 1 à 5, comprenant de plus un composant (III), dans lequel le composant (III) est une étiquette pour purification.

7. Peptide de fusion selon l'une quelconque des revendications 1 à 6, pour une utilisation en tant que médicament.

8. Composition pharmaceutique comprenant un peptide de fusion selon l'une quelconque des revendications 1 à 6 et éventuellement un véhicule pharmaceutique.

9. Utilisation d'un peptide de fusion selon l'une quelconque des revendications 1 à 6 pour la préparation d'une composition pharmaceutique destinée au traitement de maladies choisies parmi une congestion cérébrale, une congestion cérébrale ischémique, une anoxie, une ischémie, des états neurodégénératifs chroniques et une douleur neuropathique.

10. Trousse comprenant une composition pharmaceutique selon la revendication 8 et un manuel d'instructions ou une brochure d'informations avec des instructions et/ou des informations concernant l'application de la composition pharmaceutique.

11. Peptide de fusion selon l'une quelconque des revendications 1 à 6, pour une utilisation dans une méthode de traitement de maladies choisies parmi une congestion cérébrale, une congestion cérébrale ischémique, une anoxie, une ischémie, des états neurodégénératifs chroniques et une douleur neuropathique.
